# Europäisches Patentamt
# European Patent Office
# Office européen des brevets

(19)

(11) Veröffentlichungsnummer: **0 190 533**
**B1**

(12) ## EUROPÄISCHE PATENTSCHRIFT

(45) Veröffentlichungstag der Patentschrift:
**13.09.89**

(21) Anmeldenummer: **85730165.9**

(22) Anmeldetag: **09.12.85**

(51) Int. Cl.⁴: **A 61 N 5/06,** F 21 V 9/00,
G 02 B 1/10, G 02 B 5/20,
C 03 C 17/34

(54) **UV-Bräunungsgerät.**

(30) Priorität: **08.12.84 DE 3444793**

(43) Veröffentlichungstag der Anmeldung:
**13.08.86 Patentblatt 86/33**

(45) Bekanntmachung des Hinweises auf die Patenterteilung:
**13.09.89 Patentblatt 89/37**

(84) Benannte Vertragsstaaten:
**AT BE CH DE FR GB IT LI LU NL SE**

(56) Entgegenhaltungen:
**CA-A-1 069 982**
**DE-A-2 556 528**
**DE-A-2 616 892**
**DE-A-2 906 512**
**DE-A-3 123 378**
**DE-A-3 231 270**
**DE-A-3 303 795**
**DE-B-2 165 315**
**DE-C-973 177**
**FR-A-1 537 143**
**FR-A-2 343 267**
**US-A-3 978 272**

(73) Patentinhaber: **EVB- Entwicklungs- und Vertriebsgesellschaft für Bräunungsanlagen mbH, Vorwerkstrasse 9, D-3007 Gehrden 1 (DE)**

(72) Erfinder: **Lang, Gerd, Leo- Rosenblatt- Weg 9, D-3000 Hannover 91 (DE)**

(74) Vertreter: **Eitle, Werner, Dipl.- Ing., Hoffmann, Eitle & Partner Patentanwälte Arabellastrasse 4, D-8000 München 81 (DE)**

EP 0 190 533 B1

LIBER, STOCKHOLM 1989

## Beschreibung

Die Erfindung betrifft ein Bräunungsgerät, insbesondere zur Ganzkörperbräunung, mit einer UV-Strahlungsquelle für die Erzeugung von UV-Strahlen, einem vor der UV-Strahlungsquelle angeordneten Strahlenfilter sowie mit einer integrierten Turbine zur Erzeugung einer als Wärmeschutz dienenden Luftströmung zwischen dem Strahlenfilter und einem den UV-Strahlen ausgesetzten Benutzer.

Ein derartiges Solarium ist aus der DE-OS-2 906 512 bekannt. Zur Verhinderung der Wärmeübertragung von der UV-Bestrahlungsquelle auf die Haut ist bei diesem vorbekannten Gerät ein Kaltluftvorhang bzw. ein Kaltluftpolster sofort nach der Filterscheibe vorgesehen. Dieser Kaltluftvorhang wird durch ein Gebläse erzeugt.

Allgemein bekannt sind ferner Bräunungsgeräte mit einem im Abstand vor der UV-Strahlungsquelle angeordneten Blaufilter, welches bevorzugt für einen Strahlungsbereich zwischen 280 und 450 nm durchlässig ist. Unterhalb des Blaufilters ist bei diesen Bräunungsgeräten noch ein Sicherheitsglas vorgesehen, welches zwei wichtige Funktionen hat.

Ein erster Aspekt ist die Funktion des zusätzlichen Sicherheitsglases als Wärmeschutzfilter. Die von der UV-Strahlungsquelle erzeugte Strahlung besitzt nämlich auch noch nach dem Passieren des Blaufilters einen nicht unerheblichen Anteil von Wärmestrahlung, welche auf die Haut des Benutzers auftrifft. Die hierdurch bedingte Erwärmung der Haut ist zumindest unangenehm, kann bei den heute verwendeten hohen Strahlungsleistungen aber auch zu Verbrennungen führen. Das zwischen dem Blaufilter und dem Benutzer angeordnete Sicherheitsglas muß deshalb zumindest so viel Wärmestrahlung absorbieren, daß eine Verbrennung der Haut ausgeschlossen ist. Allerdings hat diese zum Schutz der Haut unerläßliche Maßnahme den gravierenden Nachteil, daß nicht nur die gewünschte Verringerung der auf die Haut auftreffenden Wärmestrahlung eintritt; vielmehr absorbiert das Sicherheitsglas in an sich unerwünschter Weise auch einen erheblichen Anteil der bräunenden UV-Strahlen. Die hierdurch bedingte Reduzierung der Strahlungsintensität um bis zu 40 % läßt sich nur durch eine entsprechende Verlängerung der Bestrahlungszeit ausgleichen.

Die zweite wichtige Funktion des Sicherheitsglases ist der Schutz des in der Regel unbekleideten Benutzers. Bei den bisher bekannten, unmittelbar vor der UV-Strahlungsquelle angeordneten und somit im Betrieb sehr heiß werdenen Blaufiltern konnte es nämlich durchaus vorkommen, daß diese zerbrechen. In diesem Fall verhindert das zusätzlich vorgesehene Sicherheitsglas, daß die heißen Scherben auf den Benutzer des Bräunungsgeräts herunterfallen.

Ein Filter zum Schutz der menschlichen Haut vor Sonnenbrand ist beispielsweise aus der FR-A-2 343 267 bzw. der auf die gleiche prioritätsbegründende Anmeldung zurückgehenden DE-A-2 609 194 bekannt. Auf einem Festkörper aus anorganischem Glas, beispielsweise Silikat- oder Quarzglas, oder aus organischem Kunststoff, beispielsweise Acrylglas oder Polyester, sind mehrere Absorptions- und/oder Reflexionsschichten aufgebracht, wobei jede Schicht in einem anderen Spektralbereich wirksam ist. Physiologisch schädliche, sog. erythemwirksame Strahlen mit Wellenlängen unterhalb 320 nm werden von dem Strahlenschutzfilter ausgeblendet. Ebenso werden Strahlen im Wellenlängenbereich über 450 nm möglichst weitgehend ausgefiltert, um den zu bestrahlenden Körper weitgehend vor zu starker Wärmebelastung zu schützen.

Nach neueren wissenschaftlichen Erkenntnissen wird das UV-Spektrum in einen sog. erythemwirksamen und einen nicht erythemwirksamen, für die menschliche Haut also unschädlichen Strahlungsanteil unterteilt. Die kritische Wellenlänge wird bei 330 nm angenommen. Oberhalb dieser Grenzwellenlänge ist eine Beeinträchtigung der menschlichen Haut so gut wie ausgeschlossen, bei Wellenlängen kleiner als 330 nm kann es im Extremfall zur Ausbildung von Karzinomen kommen. Bisher wurde allerdings ein gewisser Anteil von UVA-Strahlung unterhalb von 330 nm, mindestens im Bereich zwischen 320 und 330 nm, als zur Erzielung der gewünschten Bräunung notwendig angesehen, auch auf die Gefahr hin, daß diese kurzwellige UVA-Strahlung, jedenfalls in höheren Dosen, hautschädlich sein kann. Aus diesem Grunde ist auch in der erwähnten FR-A-2 343 267 eine untere Grenzwellenlänge von 320 nm für das darin beschriebene Strahlenschutzfilter zur Direkt-Pigmentierung der Haut bei Sonnenbestrahlung angegeben.

Mit der DE-A-3 231 270 ist ferner eine Zusatzvorrichtung für Besonnungsgeräte bekannt geworden, bei der mittels eines Gebläses Luftstrahlen aus Luftöffnungen über den auf einer Bestrahlungsliege liegenden Benutzer geblasen werden. Hierdurch soll eine Schweißentwicklung unterbunden werden.

Ausgehend von dem geschilderten Stand der Technik und zur Vermeidung der erwähnten Nachteile liegt der vorliegenden Erfindung die Aufgabe zugrunde, ein verbessertes Bräunungsgerät zu schaffen, mit dem sich bei kurzer Behandlungszeit eine tiefe Bräunung der Haut erzielen läßt, die Bestrahlung jedoch ohne schädliche Nebenwirkungen und Gefahr für den Benutzer ist.

Bei der Lösung dieser Aufgabe wird ausgegangen von einem Bräunungsgerät der eingangs beschriebenen Art; gelöst wird die Aufgabe dadurch, daß das vor der UV-Strahlungsquelle angeordnete Strahlenfilter aus spezialgehärtetem und hitzebeständigem Glas besteht und nur für Strahlung in einem Wellenlängenbereich zwischen 335 und 550 nm durchlässig ist.

Der Erfindung liegt die Erkenntnis zugrunde, daß sich ein ausgezeichneter Bräunungseffekt auch mit relativ langwelliger UV-Strahlung und unter völligem Verzicht auf kurzwellige UVA-Strahlung im Bereich unter 335 nm erzielen läßt, wenn man gleichzeitig - sozusagen am anderen Ende des Spektrums - einen gewissen Anteil von kurzwelligem sichtbaren Licht hinzunimmt. Das Strahlenfilter mit dem erfindungsgemäßen Durchlaßbereich garantiert somit eine absolute Hautverträglichkeit bei hervorragendem Bräunungseffekt.

Da das Strahlenfilter aus spezialgehärtetem und hitzebeständigem Glas besteht, kann es trotz seiner Anordnung unmittelbar vor der UV-Strahlungsquelle nicht zerbrechen, so daß eine Gefährdung des Benutzers durch herabfallende heiße Glasscherben ausgeschlossen ist. Auf das Vorsehen eines besonderen Sicherheitsglases zwischen dem Benutzer und dem Strahlenfilter kann deshalb verzichtet werden, so daß die volle Strahlungsintensität in dem vom Strahlenfilter durchgelassenen Spektralbereich zur Bräunung zur Verfügung steht. Eine örtliche Überhitzung der bestrahlten Haut des Benutzers wird durch die als Wärmeschutz dienende Luftströmung verhindert.

In vorteilhafter Weiterbildung des erfindungsgemäßen Bräunungsgeräts sind zwei seitliche Stützen vorgesehen, zwischen denen sich oben eine die UV-Strahlungsquelle enthaltende Strahlungseinheit und unten eine Liegeeinheit für den Benutzer befindet. Eine der Stützen ist hohl ausgebildet und weist in ihrem Inneren Leitbleche auf. An ihrer Innenseite ist eine Luftaustrittsöffnung vorgesehen. Bevorzugt ist an der Innenseite der gegenüberliegenden Stütze eine Lufteintrittsöffnung vorgesehen. Die zweckmäßig innerhalb der Liegeeinheit angeordnete Turbine erzeugt eine Luftströmung, die durch die Luftaustrittsöffnung in der einen Stütze in den Bereich über der Liegeeinheit ausgeblasen und über die Lufteintrittsöffnung der gegenüberliegenden Stütze angesaugt wird, so daß ein Luftströmungskreislauf entsteht. Diese Luftströmung führt die Wärme von der Hautoberfläche des Benutzers wirksam ab, so daß trotz der hohen Strahlungsintensität die Gefahr von Verbrennungen ausgeschlossen ist.

Die Kühlung durch die Luftströmung kann mittels einer Klimaanlage, welche vorzugsweise ebenfalls innerhalb der Liegeeinheit angeordnet ist, noch intensiviert werden.

Besonders bewährt haben sich Strahlenfilter, die aus spezialgehärtetem und hitzebeständigem Glas als Substrat bestehen, auf welchem mehrere dichroitische Schichten aufgebracht sind. Derartige Strahlenfilter sind an sich bereits bekannt, und zwar als Farbeffektfilter beim Theater zur Erzeugung besonderer Lichteffekte. Geeignet zur Herstellung eines solchen Strahlenfilters ist beispielsweise das unter der Bezeichnung "Tempax" im Handel erhältliche Glas, welches auch unter großer Hitzeeinwirkung nicht bricht.

Insbesondere bei Bräunungsgeräten zur Ganzkörperbräunung ist es zweckmäßig, das Strahlungsfilter aus mehreren, direkt nebeneinander angeordneten Filtergläsern zusammenzusetzen. Auf diese Weise läßt sich ein Strahlenfilter bauen, welches sich über den gesamten Bereich der Strahlungseinheit erstreckt. Um ein gutes Aneinanderliegen der einzelnen Filtergläser ohne metallene Stege zu erreichen, sind die jeweils benachbarten Filtergläser an ihren einander zugewandten Seiten mit einem treppenförmigen Versatz versehen, so daß sie stumpf aneinander liegen.

Anstelle einer oberen Strahlungseinheit, die auf zwei seitliche Stützen gelagert ist, kann bei einer weiteren Ausführungsform des erfindungsgemäßen Bräunungsgeräts die Strahlungseinheit dachförmig ausgebildet und mittels einer Hubvorrichtung nach oben oder unten bewegbar sein. Die Erzeugung der Luftströmung zwischen dem Strahlenfilter und dem Benutzer bildet sich dann von der oberen Strahlungseinheit in Richtung auf die darunter befindliche Liegeeinheit aus. Hierzu sind zweckmäßigerweise an der in der unteren Liegeeinheit angeordneten Turbine Luftschläuche angeordnet, die zur oberen Strahlungseinheit führen.

Eine besonders gleichmäßige Bräunung bei gleichzeitig guter Ausnutzung der von der UV-Strahlungsquelle abgegebenen Strahlungsleistung ergibt sich, wenn ein ganzflächiger, sich über die gesamte Strahlungseinheit erstreckender Reflektor hinter der UV-Strahlungsquelle vorgesehen ist.

Ausführungsbeispiele der Erfindung werden nachstehend anhand der beigefügten Zeichnungen näher erläutert. Es zeigen:

Fig. 1    ein Bräunungsgerät für die Ganzkörperbräunung, in einer vereinfachten perspektivischen Ansicht;

Fig. 2    eine prinzipielle Darstellung zur Verdeutlichung der Wirkungsweise des Bräunungsgeräts von Fig. 1, von der Stirnseite aus gesehen;

Fig. 3    eine prinzipielle Darstellung der Wirkungsweise des Bräunungsgeräts von Fig. 1, von der Seite her gesehen;

Fig. 4    eine Fig. 2 entsprechende prinzipielle Darstellung eines Bräunungsgeräts mit zusätzlichen Ansaugöffnungen an den Längsseiten der Liegeeinheit;

Fig. 5    die Strahlungseinheit des Bräunungsgeräts von Fig. 1, von unten;

Fig. 6    die Strahlungseinheit gemäß Fig. 5, in einem Schnittbild;

Fig. 7    ein Detail der Strahlungseinheit gemäß Fig. 5;

Fig. 8    eine zweite Ausführungsform eines Bräunungsgeräts, in vereinfachter perspektivischer Darstellung.

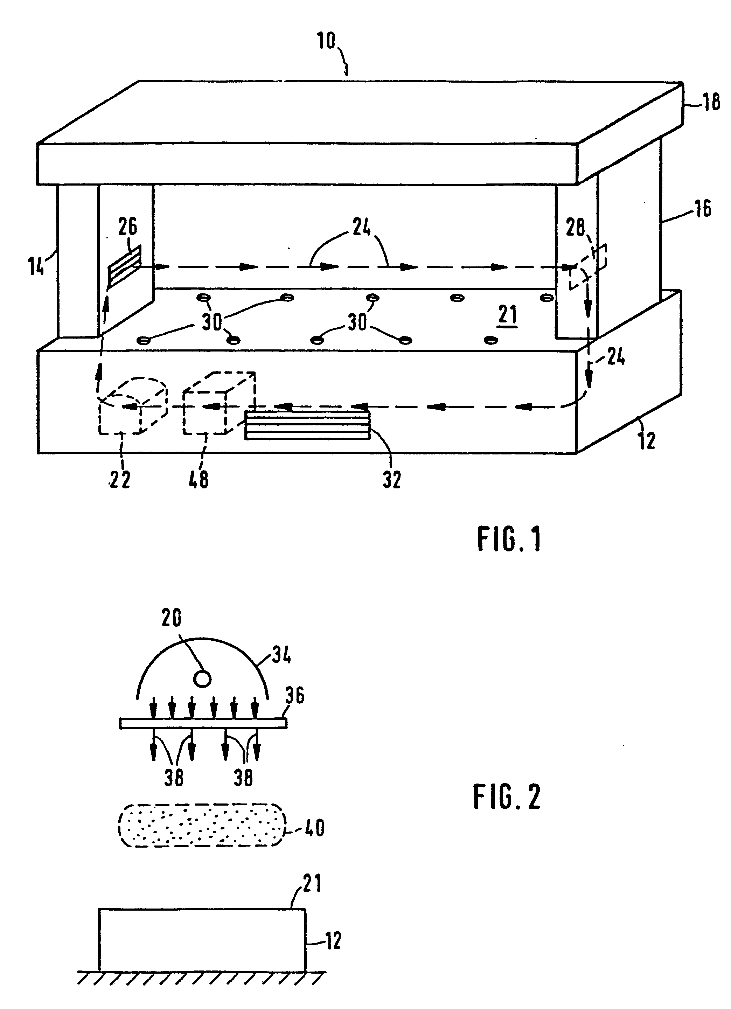

Das in Fig. 1 insgesamt mit 10 bezeichnete Bräunungsgerät besteht aus einer Liegeeinheit 12, zwei seitlich angeordneten Stützen 14 und 16 sowie einer oberen Strahlungseinheit 18.

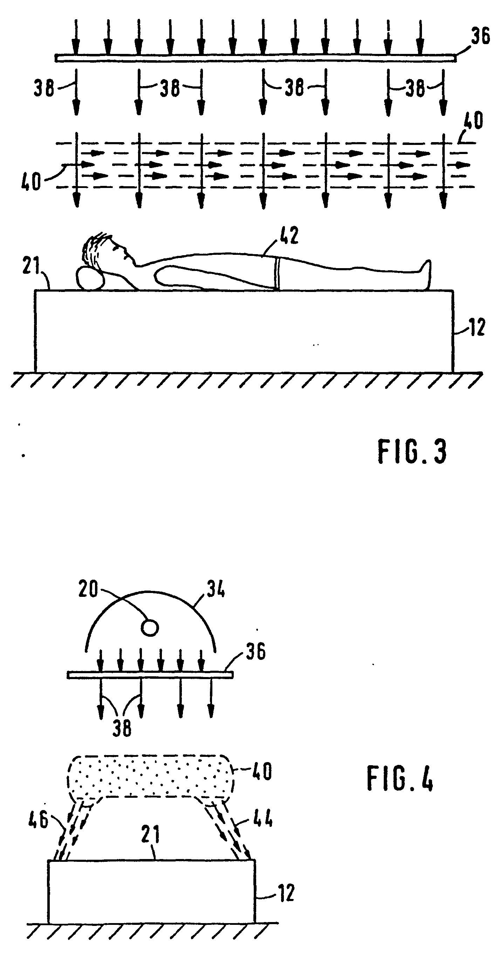

Wie aus den Fig. 2 und 4 ersichtlich, enthält die obere Strahlungseinheit 18 eine UV-Strahlungsquelle 20 und einen dieser zugeordneten ganzflächigen Reflektor 34. Fig. 3 verdeutlicht, daß ein Benutzer 42 auf einer Liegefläche 21 der Liegeeinheit 12 Platz findet, um die Haut zu bräunen.

Innerhalb der weitgehend geschlossen ausgebildeten Liegeeinheit 12 ist eine Turbine 22 und eine Klimaanlage 48 angeordnet (vgl. Fig. 1). In der einen Seitenwand der Liegeeinheit 12 ist im unteren Bereich ein Lüftungsgitter 32 vorgesehen, so daß die Turbine 22 Luft von außen ansaugen kann.

Die seitliche Stütze 14 ist an ihrer Innenseite mit einer Luftaustrittsöffnung 26 versehen, während die andere seitliche Stütze 16 an ihrer gegenüberliegenden Innenseite eine Lufteintrittsöffnung 28 aufweist. Zumindest in ihrem unteren Bereich sind die beiden Stützen 14 und 16 soweit hohl ausgebildet, daß sich eine von der Turbine 22 erzeugte Luftströmung 24 darin ausbilden kann. Innerhalb der Stützen 14 und 16 sind Umlenkeinrichtungen in Form von (nicht dargestellten) Leitblechen vorgesehen, welche bewirken, daß die Luftströmung 24 aus der Luftaustrittsöffnung 26 austritt und an der gegenüberliegenden Seite durch die Lufteintrittsöffnung 28 wieder angesaugt wird.

Wie in den Fig. 2, 3 und 4 erkennbar, bildet die Luftströmung 24 eine Art Kaltluftpolster 40 zwischen der UV-Strahlungsquelle 20 und der unteren Liegeeinheit 12, welche den daraufliegenden Benutzer 42 (vgl. Fig. 3) vor übermäßiger Wärmeeinwirkung schützt.

Die Turbine 22 erzeugt nicht nur die Luftströmung 24, sondern sie saugt zugleich auch über die Lufteintrittsöffnung 28 in der Stütze 16 die Luft wieder an, so daß sich ein Luftströmungskreislauf ausbildet. Bei Bedarf kann die Luftströmung 24 mittels der Klimaanlage 48 gekühlt werden.

Gemäß der schematischen Darstellung in Fig. 2 besteht die UV-Strahlungsquelle 20 aus einer senkrecht zur Zeichenebene angeordneten, rohrförmig ausgebildeten UV-Lampe, welche teilweise von dem Reflektor 34 umgeben ist. Die von der UV-Strahlungsquelle 20 ausgesandte UV-Strahlung 38 wird vorzugsweise nach unten in Richtung auf die Liegefläche 21 der Liegeeinheit 12 abgestrahlt.

Direkt unterhalb der UV-Strahlungsquelle 20 ist ein Strahlenfilter 36 angeordnet, das nur für Strahlung in einem Wellenlängenbereich zwischen 335 und 550 nm durchlässig ist. Dieser Bereich umfaßt langwellige UVA-Strahlung und kurzwelliges sichtbares Licht. Das Strahlenfilter 36 besteht aus spezialgehärtetem und hitzebeständigem Glas als Substrat, auf dem mehrere dichroitische Schichten aufgebracht sind. Als Strahlenfilter kann beispielsweise ein von der Fa. Schott Glaswerke, Mainz, unter der Typen-Nr. 912 hergestelltes optisches Filter Verwendung finden, das aus hitzebeständigem "Tempax"-Glas als Substrat besteht.

Durch das von der Luftströmung 24 gebildete Kaltluftpolster 40 kann die vom Strahlenfilter 36 durchgelassene UV-Strahlung 38 ungehindert auf die Haut des Benutzers 42 treffen. Das Kaltluftpolster 40 dient ausschließlich als Wärmeschutz, um ein Verbrennen der Haut zu verhindern, während ein Strahlungsverlust bzw. Bräunungsverlust nicht eintritt.

Bei dem Ausführungsbeispiel gemäß Fig. 4 wird das Kaltluftpolster 40 oberhalb der Liegefläche 21 noch durch zwei seitliche Luftströmungen 44 und 46 ergänzt, so daß ein auf der Liegefläche 21 liegender Benutzer 42 haubenartig von einem Luftpolster umhüllt ist. Die beiden seitlichen Luftströmungen 44 und 46 können sich ausbilden, weil an den beiden Längsseiten der Liegefläche 21 mehrere zusätzliche Ansaugöffnungen 30 vorgesehen sind, durch welche ein Teil der aus der Luftaustrittsöffnung 26 austretenden Kühlluft von der Turbine 22 angesaugt wird (vgl. Fig. 1).

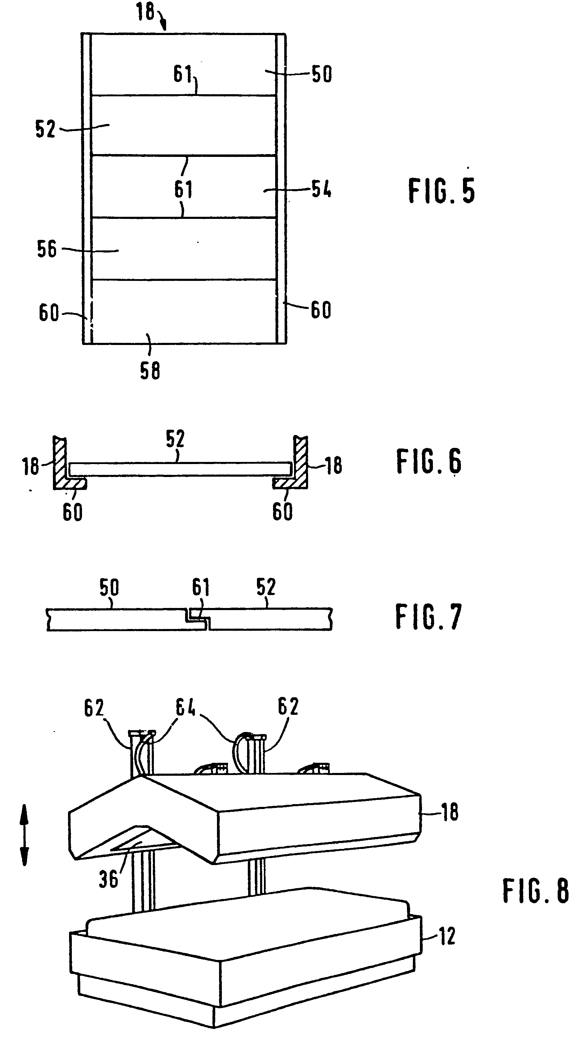

In Fig. 5 ist die obere Strahlungseinheit 18 in einer Ansicht dargestellt, wie sie sich dem auf der Liegeeinheit 12 befindlichen Benutzer 42 darstellt. Die gesamte Unterseite der Liegeeinheit 12 ist mit mehreren nebeneinander angeordneten Filtergläsern 50 - 58 abgedeckt, welche gemeinsam das Stahlenfilter 36 bilden.

Aus dem Querschnitt in Fig. 6 ist erkennbar, daß die einzelnen Filtergläser 50 - 58 auf zwei gegenüberliegenden seitlichen Kanten 60 der Strahlungseinheit 18 aufliegen und somit gehalten sind. Die nebeneinander liegenden Filtergläser 50, 52 liegen ohne Verwendung besonderer Rahmenteile stumpf aneinander. Dies wird, wie die Detailansicht gemäß Fig. 7 zeigt, dadurch erreicht, daß die Filtergläser 50, 52 einen treppenförmigen Versatz 61 aufweisen und somit ineinandergreifen. Hierdurch gelingt es, über den gesamten Bereich der Strahlungseinheit 18 ein durchgehendes flächiges Strahlenfilter 36 zu realisieren.

Bei der in Fig. 8 dargestellten alternativen Ausführungsform eines Bräunungsgeräts ist die obere Strahlungseinheit 18 dachförmig ausgebildet. Anstelle der seitlichen Stützen 14, 16 (vgl. Fig. 1) sind hier für die Halterung der Strahlungseinheit 18 zwei Hubvorrichtungen 62 vorgesehen, mit denen die Strahlungseinheit 18 nach oben und nach unten zwecks Einstellung eines optimalen Abstands zur Liegeeinheit 12 bewegt werden kann. Die als Wärmeschutz dienende Luftströmung wird hier dadurch erzeugt, daß Luft von oben von der Strahlungseinheit 18 her nach unten auf die Liegeeinheit 12 geblasen wird. Zu diesem Zweck sind Luftschläuche 64 vorgesehen, welche mit der in der Liegeeinheit 12 eingebauten (nicht dargestellten) Turbine in Verbindung stehen, und welche die Luft zu seitlich des Strahlenfilters 36 angeordneten Austrittsöffnungen leiten. Auch wenn die dadurch ausgebildete Luftströmung hier nicht waagerecht, sondern senkrecht nach unten ausgerichtet ist, wird gleichwohl ein wirksamer Wärmeschutz durch die strömende Luft gebildet.

**Patentansprüche**

1. Bräunungsgerät (10), insbesondere zur Ganzkörperbräunung, mit
- einer UV-Strahlungsquelle (20) für die Erzeugung von UV-Strahlen,
- einem vor der UV-Strahlungsquelle (20) angeordneten Strahlenfilter (36),
- einer integrierten Turbine (22) zur Erzeugung einer als Wärmeschutz dienenden Luftströmung (24) zwischen dem Strahlenfilter (36) und einem den UV-Strahlen ausgesetzten Benutzer;
dadurch gekennzeichnet, daß das Strahlenfilter (36)
- aus spezialgehärtetem und hitzebeständigem Glas besteht und
- nur für Strahlung in einem Wellenlängenbereich zwischen 335 und 550 nm durchlässig ist.

2. Bräunungsgerät nach Anspruch 1,
dadurch gekennzeichnet, daß zwei seitliche Stützen (14, 16) vorgesehen sind, zwischen denen sich oben eine die UV-Strahlungsquelle (20) enthaltende Strahlungseinheit (18) und unten eine Liegeeinheit (12) für den Benutzer (42) befindet, und daß die Turbine (22) innerhalb der Liegeeinheit (12) angeordnet ist.

3. Bräunungsgerät nach Anspruch 2,
dadurch gekennzeichnet, daß mindestens eine Stütze (14) an ihrer Innenseite eine Luftaustrittsöffnung (26) für die von der Turbine (22) erzeugte Luftströmung (24) besitzt, und daß die Stütze (14) hohl ausgebildet ist und in ihrem Inneren Leitbleche zur Führung der Luftströmung (24) aufweist.

4. Bräunungsgerät nach einem der Ansprüche 2 bis 3,
dadurch gekennzeichnet, daß an der Innenseite der einen Stütze (14) eine Luftaustrittsöffnung (26) und an der Innenseite der gegenüberliegenden Stütze (16) eine Lufteintrittsöffnung (28) vorgesehen ist, durch welche Luft in die hohl ausgebildete Stütze (16) gesaugt wird.

5. Bräunungsgerät nach Anspruch 4,
dadurch gekennzeichnet, daß die die Luftströmung (24) erzeugende Turbine (22) die Luft über die Lufteintrittsöffnung (28) ansaugt, derart, daß ein Luftströmungskreislauf entsteht.

6. Bräunungsgerät nach Anspruch 4 oder 5,
dadurch gekennzeichnet, daß an den beiden Längsseiten der auf der Liegeeinheit (12) befindlichen Liegefläche (21) zusätzliche Ansaugöffnungen (30) vorgesehen sind.

7. Bräunungsgerät nach einem der Ansprüche 1 bis 6,
dadurch gekennzeichnet, daß eine Klimaanlage (48) vorgesehen ist, um die Luftströmung (24) zu kühlen.

8. Bräunungsgerät nach Anspruch 7,
dadurch gekennzeichnet, daß die Klimaanlage (48) innerhalb der Liegeeinheit (12) angeordnet ist.

9. Bräunungsgerät nach einem der Ansprüche 1 bis 8,
dadurch gekennzeichnet, daß das Strahlenfilter (36) aus spezialgehärtetem und hitzebeständigem Glas als Substrat besteht, auf welchem mehrere dichroitische Schichten aufgebracht sind.

10. Bräunungsgerät nach einem der Ansprüche 1 bis 9,
dadurch gekennzeichnet, daß das Strahlenfilter (36) aus mehreren, direkt nebeneinander angeordneten Filtergläsern (50 - 58) zusammengesetzt ist und sich über den Bereich der Strahlungseinheit (18) erstreckt.

11. Bräunungsgerät nach Anspruch 10,
dadurch gekennzeichnet, daß die jeweils benachbarten Filtergläser (50 - 58) an ihren einander zugewandten Seiten einen treppenförmigen Versatz (61) aufweisen und stumpf aneinander liegen.

12. Bräunungsgerät nach Anspruch 1,
dadurch gekennzeichnet, daß die Strahlungseinheit (18) dachförmig ausgebildet ist und mittels einer Hubvorrichtung (62) nach oben bzw. unten bewegbar ist.

13. Bräunungsgerät nach Anspruch 12,
dadurch gekennzeichnet, daß die Turbine (22) über Luftschläuche (64) eine von der Strahlungseinheit (18) bzw. von der UV-Strahlungsquelle (20) in Richtung auf die darunter befindliche Liegeeinheit (12) gerichtete Luftströmung erzeugt.

14. Bräunungsgerät nach einem der Ansprüche 1 bis 13,
dadurch gekennzeichnet, daß ein ganzflächiger, sich über die gesamte Strahlungseinheit (18) erstreckender Reflektor (34) hinter der UV-Strahlungsquelle (20) vorgesehen ist.

**Claims**

1. Tanning apparatus (10), in particular for whole body tanning, with
- a UV radiation source (20) for producing UV radiation,
- a radiation filter (36) arranged in front of the UV radiation source (20),
- an integrated turbine (22) for producing an air stream (24) serving as heat protection between the radiation filter (36) and a user subjected to the UV radiation;
- characterised, in that radiation filter (36) consists of
- specially hardened and heat resistant glass and
- is permeable only to radiation at a wavelength of between 335 and 550 nm.

2. Tanning apparatus according to claim 1,
characterised in that two lateral supports (14, 16) are provided between which there is located at the top a UV radiation unit (18) containing the UV radiation source (20) and a bed unit (12) for the user (42) at the bottom, and the turbine (22) is arranged within the bed unit (12).

3. Tanning apparatus according to claim 2,
characterised in that the inner side of at least one support (14) has an air discharge opening (26) for the air stream (24) produced by the turbine (22), and that the support (14) is hollow and its interior has guide surfaces for guiding the air stream (24).

4. Tanning apparatus according to one of the claims 2 to 3,

characterised in that on the inner side of one of the supports (14) there is provided an air discharge opening (26) and on the inner side of the oppositely arranged support (16) there is provided an air inlet opening (28) through which air is sucked into the hollow support (16).

5. Tanning apparatus according to claim 4,

characterised in that the turbine (22) generating the air stream (24) sucks in the air through the air intake opening (28) in such a way as to produce a circulating air stream.

6. Tanning apparatus according to claim 4 or 5,

characterised in that additional suction openings (30) are provided in the two longitudinal sides of the lying-down surface (21) situated on the bed unit (12).

7. Tanning apparatus according to one of the claims 1 to 6,

characterised in that an air conditioning plant (48) is provided in order to cool the air stream (24).

8. Tanning apparatus according to claim 7,

characterised in that the air conditioning plant (48) is arranged within the bed unit (12).

9. Tanning apparatus according to one of the claims 1 to 8,

characterised in that the radiation filter (36) consists of specially hardened and heat resistant glass in the form of a substrate, to which are applied several dichroic layers.

10. Tanning apparatus according to one of the claims 1 to 9,

characterised in that the radiation filter (36) is composed of several filter glasses (50 - 58) arranged directly beside each other and which extends over the area of the radiation unit (18).

11. Tanning apparatus according to claim 10,

characterised in that each of the adjacent filter glasses (50 - 58) have their mutually facing side edges offset in step-like manner (61) and arranged in abutment with each other.

12. Tanning apparatus according to claim 1,

characterised in that the radiation unit (18) is roof-shaped and movable upwards or downwards by means of a lifting device (62).

13. Tanning apparatus according to claim 12,

characterised in that the turbine (22) produces an airstream which is directed via air hoses (64) from the radiation unit (18) or the UV radiation source (20) in the direction of the bed unit (12) situated beneath the UV radiation source (20).

14. Tanning apparatus according to one of the claims 1 to 13,

characterised in that a one-surface reflector (34) extending over the entire radiation unit (18) is provided behind the UV radiation source (20).

## Revendications

1. Appareil de bronzage (10), en particulier pour le bronzage du corps entier, comportant
   - une source de rayonnement UV (20) pour produire des rayons UV,
   - un filtre à rayonnement (36), disposé devant la source de rayonnement UV (20),
   - une turbine intégrée (22) pour produire un écoulement d'air (24) servant de protection contre la chaleur entre le filtre à rayonnement (36) et un utilisateur exposé aux rayons UV,

   caractérisé en ce que le filtre à rayonnement (36)
   - est constitué d'un verre trempé spécial et résistant à la chaleur, et
   - n'est transparent qu'au rayonnement dont la longueur d'onde est comprise entre 335 et 550 nm.

2. Appareil de bronzage selon la revendication 1, caractérisé en ce qu'il est prévu deux appuis latéraux (14, 16), entre lesquels se trouvent en haut une unité de rayonnement (18) contenant la source de rayonnement UV (20), et en bas un support de table (12) pour l'utilisateur (42), et que la turbine (22) est installée à l'intérieur du support de table (12).

3. Appareil de bronzage selon la revendication 2, caractérisé en ce qu'au moins un appui (14) possède, sur sa face intérieure, un orifice de sortie d'air (26) pour l'écoulement d'air (24) produit par la turbine (22), et que l'appui (14) est creux et, dans son volume intérieur, possède des chicanes destinées au guidage de l'écoulement d'air (24).

4. Appareil de bronzage selon l'une des revendications 2 ou 3, caractérisé en ce qu'il est prévu sur le côté intérieur de l'un des montants (14) un orifice de sortie d'air (26), et sur le côté intérieur du montant en regard (16) un orifice d'entrée d'air (28), par lequel de l'air est aspiré dans l'appui creux (16).

5. Appareil de bronzage selon la revendication 4, caractérisé en ce que la turbine (22) produisant l'écoulement d'air (24) aspire l'air par l'orifice d'entrée d'air (28) de façon qu'il se crée un écoulement d'air en circuit fermé.

6. Appareil de bronzage selon la revendication 4 ou 5, caractérisé en ce qu'il est prévu sur les deux grands côtés de la table (21) se trouvant sur le support de table (12) des orifices d'aspiration supplémentaires (30).

7. Appareil de bronzage selon l'une des revendications 1 à 6, caractérisé en ce qu'il est prévu une installation de climatisation (48) pour refroidir l'écoulement d'air (24).

8. Appareil de bronzage selon la revendication 7, caractérisé en ce que l'installation de climatisation (48) est disposée à l'intérieur du support de table (12).

9. Appareil de bronzage selon l'une des revendications 1 à 8, caractérisé en ce que le filtre à rayonnement (36) est constitué d'un verre trempé spécial et résistant à la chaleur, servant de substrat sur lequel sont appliquées plusieurs couches dichroïques.

10. Appareil de bronzage selon l'une des revendications 1 à 9, caractérisé en ce que le filtre à rayonnement (36) est composé de plusieurs verres filtrants (50 - 58) directement disposés les uns à côté des autres, et s'étend sur toute la surface de l'unité de rayonnement (18).

11. Appareil de bronzage selon la revendication 10, caractérisé en ce que les paires voisines de verres filtrants (50 - 58) comportent, sur leurs faces en regard, un épaulement étagé (61), et s'appuient l'un contre l'autre en aboutement.

12. Appareil de bronzage selon la revendication 1, caractérisé en ce que l'unité de rayonnement (18) a la forme d'un toit et peut être déplacée vers le haut ou vers le bas à l'aide d'un dispositif de levage (62).

13. Appareil de bronzage selon la revendication 12, caractérisé en ce que la turbine (22) produit, par l'intermédiaire de tuyaux flexibles d'air (64), un écoulement d'air dirigé, à partir de l'unité de rayonnement (18) ou de la source de rayonnement UV (20), dans la direction du support de table (12) situé en-dessous.

14. Appareil de bronzage selon l'une des revendications 1 à 13, caractérisé en ce qu'il est prévu en arrière de la source de rayonnement UV (20) un réflecteur (34) de grande surface, s'étendant sur la totalité de l'unité de rayonnement (18).

10

18

26

24

28

16

14

30

30

21

24

22

48

32

12

**FIG. 1**

20

34

36

38    38

40

21

12

**FIG. 2**

1

36

38

40

40

42

21

12

**FIG. 3**

20

34

36

38

40

46

44

21

12

**FIG. 4**

3

18

61

52

61

54

50

56

60

60

58

**FIG.5**

18 — 52 — 18

60 — 60

**FIG.6**

50 — 61 — 52

**FIG.7**

62 — 64 — 62

18

36

12

**FIG.8**